# EUROPEAN PATENT APPLICATION

(11) **EP 2 674 481 A1**
(43) Date of publication of application: **18.12.2013**
(21) Application number: 13170633.5
(22) Date of filing: 05.06.2013
(51) Int. Cl.: C12M 1/33, C12M 1/00

(54) **Cell isolation apparatus**

(30) Priority: 12.06.2012 JP 2012133090
(71) Applicant: Nihon Kohden Corporation, Shinjuku-ku Tokyo (JP)
(72) Inventor: Shioyama, Takahiro, Shinjuku-ku, Tokyo (JP); Suzuki, Akane, Shinjuku-ku, Tokyo (JP); Kubo, Hirotsugu, Shinjuku-ku, Tokyo (JP)
(74) Representative: Grünecker, Kinkeldey, Stockmair & Schwanhäusser

(57) **Abstract**

A cell isolation apparatus in which tissue and fluid that are accommodated in a container are pipetted by a pipette, and cells are isolated from the tissue, the cell isolation apparatus includes: a nozzle to which the pipette is attached; a pump which is connected to the nozzle; a controller which is configured to control an operation of the pump to cause air to be ejected from the nozzle or to be sucked into the nozzle, thereby causing the pipette to perform pipetting; and a condition inputting unit in which a user inputs a condition of control of the controller,

## Description

### CROSS-REFERENCE TO RELATED APPLICATION(S)

This application is based upon and claims the benefit of priority from prior Japanese patent application No. 2012-133090, filed on June 12, 2012, the entire contents of which are incorporated herein by reference.

### BACKGROUND

The presently disclosed subject matter relates to a cell isolation apparatus which can be used in the case where a pathological analysis, a regenerative medical process, or the like is performed.

A rapid analysis on a tissue slice is performed by a cytotechnologist or a pathologist. It is often that a specimen formed with a frozen slice is incompletely produced. Moreover, some cases are difficult to be diagnosed based on a tissue slice in which only one cross section can be observed. To comply with this, after cells in tissue are isolated, the cells in the tissue are analyzed by a flow cytometer, so that the cells can be thoroughly analyzed, and a result of the analysis may be contributory to a more correct diagnosis. In cell isolation from tissue, a plurality of steps such as mincing of the tissue and filtering, a skilled technique for stable analysis, and restraint of a technical expert for a predetermined time period are required. Therefore, it has been requested to automatize the procedures ranging from cell isolation to measurement.

Japanese Patent No. 4156847 discloses a device for mincing tissue required for culturing cell. The device includes a cylinder member in which a metal meshes is disposed in each of upper and lower openings. A tissue piece is placed on the metal mesh, and a process of centrifugal separation is performed on the cylinder member, thereby mincing the tissue.

According to the configuration disclosed in Japanese Patent No. 4156847, the mincing process can be automatically performed, but procedures such as piece preparation and staining cannot be automatized. Moreover, it is not considered that a cell suspension containing isolated cells is automatically recovered.

### SUMMARY

The presently disclosed subject matter may provide a technique by which a certain result of cell isolation can be automatically obtained without performing a procedure that depends on the skill level.

There may be provided a cell isolation apparatus in which tissue and fluid that are accommodated in a container are pipetted by a pipette, and cells are isolated from the tissue, the cell isolation apparatus comprising: a nozzle to which the pipette is attached; a pump which is connected to the nozzle; a controller which is configured to control an operation of the pump to cause air to be ejected from the nozzle or to be sucked into the nozzle, thereby causing the pipette to perform pipetting; and a condition inputting unit in which a user inputs a condition of control of the controller, as the condition of the control, at least one of a rate of ejection of air from the nozzle, a rate of suction of air into the nozzle, an amount of suction of air, an amount of ejection of air, a duration time of ejection of air, a duration time of suction of air, an interval between ejection and suction, and ejection and suction numbers being variable.

The cell isolation apparatus may further comprise a pressure sensor which is configured to sense an internal pressure of the pipette through the nozzle.

The controller may change the condition of the control based on the internal pressure which is sensed by the pressure sensor.

The cell isolation apparatus may further comprise a treatment solution accommodator which is configured to accommodate a cell treatment solution, and the controller may control the operation of the pump to eject the cell treatment solution which is accommodated in the treatment solution accommodator, from the nozzle.

The cell isolation apparatus may further comprise a recovered solution accommodator which is configured to accommodate a cell suspension, and the controller may control the operation of the pump to cause a cell suspension which is obtained by the pipetting, to be recovered from the nozzle, and the recovered cell suspension to be accommodated in the recovered solution accommodator.

The cell isolation apparatus may further comprise a temperature regulator which is configured to regulate a temperature of the vicinity of the supporting portion, and the controller may control an operation of the temperature regulator based on a temperature which is sensed by the temperature sensor.

The cell isolation apparatus may further comprise a light-shielding member which covers the container supported by the supporting portion.

The cell isolation apparatus may further comprise a temperature sensor which is disposed in a vicinity of the supporting portion.

The cell isolation apparatus may further comprise a temperature regulator which is configured to regulate a temperature of the vicinity of the supporting portion, and the controller may control an operation of the temperature regulator based on a temperature which is sensed by the temperature sensor.

### BRIEF DESCRIPTION OF THE DRAWINGS

Fig. 1 is a perspective view showing the appearance of a cell isolation apparatus of an embodiment of the presently disclosed subject matter.
Fig. 2 is a perspective view enlargedly showing a container housing of the cell isolation apparatus.
Fig. 3 is a functional block diagram diagrammatically showing the internal configuration of the cell isolation apparatus.
Fig. 4 is an exploded perspective view showing the configuration of a cell isolation device which is to be attached to the cell isolation apparatus.
Fig. 5 is a perspective view showing the container housing in a state where a container accommodating tissue is attached to the chamber.
Figs. 6A and 6B are longitudinal sectional views each showing a state where a pipetting process is performed by using the cell isolation apparatus.
Fig. 7 is a longitudinal sectional view showing a state where a cell suspension is recovered by using the cell isolation apparatus.

### DETAILED DESCRIPTION OF EXEMPLARY EMBODIMENTS

Hereinafter, a cell isolation apparatus 100 of an embodiment of the presently disclosed subject matter will be described in detail with reference to the accompanying drawings. In the drawings, the scale is adequately changed in order to draw components in a recognizable size.

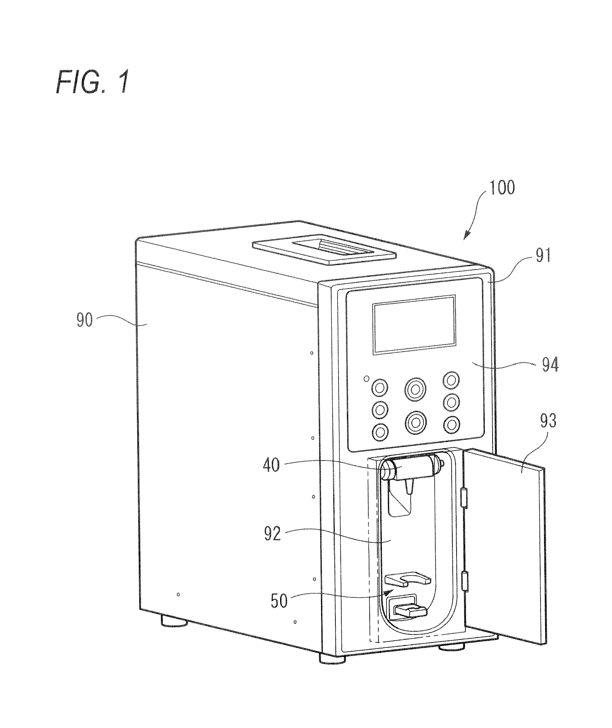

In the cell isolation apparatus 100, as shown in Fig. 1, a front panel 91 is attached to a body housing 90, so that the apparatus has a box-like appearance. A part of the front panel 91 is recessed toward the interior of the body housing 90 to form a container housing 92. A nozzle 40 and a supporting portion 50 are placed in the container housing 92.

A light-shielding cover 93 which closes and opens the container housing 92 is disposed on the lateral side of the container housing 92. When the light-shielding cover 93 is closed, light can be blocked from entering the container housing 92 from the outside.

An operating unit 94 which includes a display and a button group is disposed above the container housing 92. The operating unit 94 is used for setting and checking operations of various sections of the cell isolation apparatus 100.

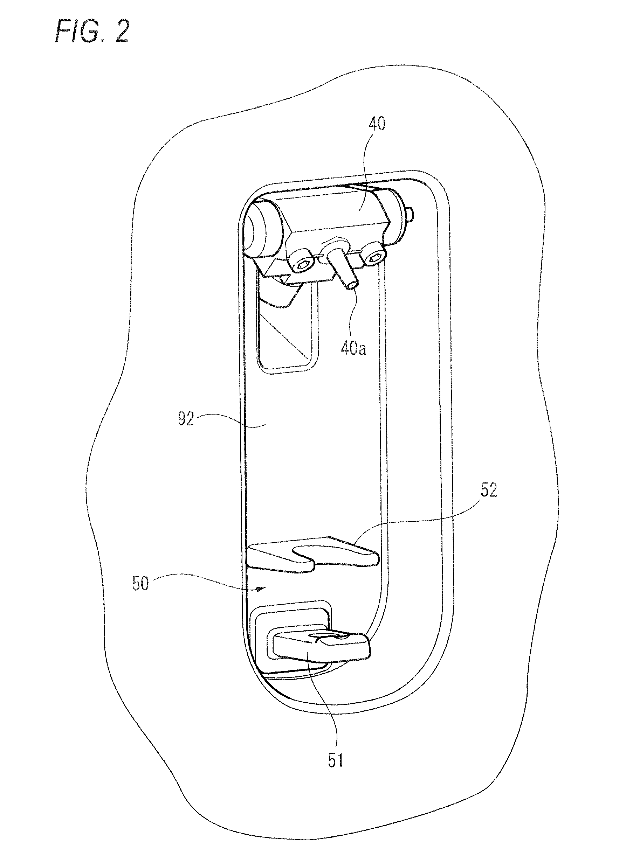

Fig. 2 enlargedly shows the configuration of the container housing 92. The nozzle 40 is placed in an upper portion of the container housing 92. The nozzle 40 is swingable in the anteroposterior direction of the cell isolation apparatus 100. Fig. 2 shows the state where the nozzle 40 is swung so that an opening 40a formed in the tip end of the nozzle is directed to the front.

The supporting portion 50 is placed in a lower portion of the container housing 92. The supporting portion 50 includes a supporting table 51 and a holder 52, and is used for supporting a cell isolation device 1 which will be described later.

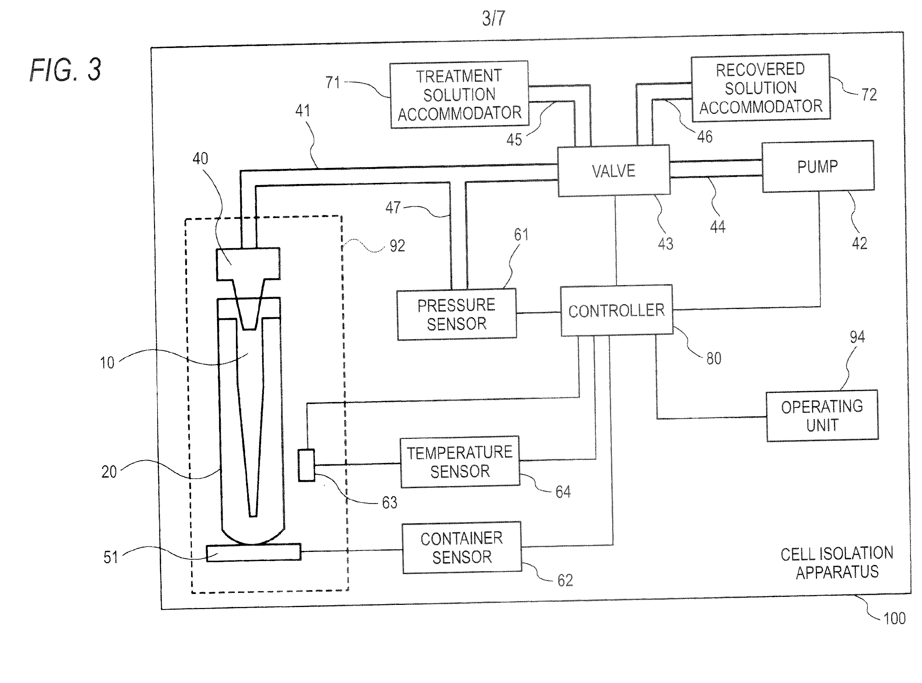

Fig. 3 is a functional block diagram showing the internal configuration of the cell isolation apparatus 100. A pump 42 is placed in the body housing 90. The nozzle 40 is connected to a valve 43 through a passage 41. The pump 42 and the valve 43 are configured in a related-art manner, and their detailed description is omitted.

Furthermore, a treatment solution accommodator 71 and a recovered solution accommodator 72 are disposed in the body housing 90. A cell treatment solution 31 which will be described later is accommodated in the treatment solution accommodator 71. The recovered solution accommodator 72 is a space for accommodating a cell suspension 33 which is recovered, and which will be described later. The treatment solution accommodator 71 is connected to the valve 43 through a first branch passage 45. The recovered solution accommodator 72 is connected to the valve 43 through a second branch passage 46.

A controller 80 is a computation processing circuit including a calculating device such as a CPU, and memories such as a RAM and a ROM, and controls the overall operation of the cell isolation apparatus 100. Various operations and functions of the controller 80 which will be hereinafter described can be realized by the operation of hardware such as circuit devices, that of software such as programs stored in an arithmetic device, or a combination of these operations.

The controller 80 is communicatably connected to the pump 42 and the valve 43. The controller 80 controls the operation of the pump 42 to set the passage 41 to a pressurized condition or a depressurized condition. The controller 80 controls the operation of the valve 43 to selectively switch among a state where the first branch passage 45 communicates with the passage 41, a state where the second branch passage 46 communicates with the passage 41, and a state where the both passages do not communicate with the passage 41.

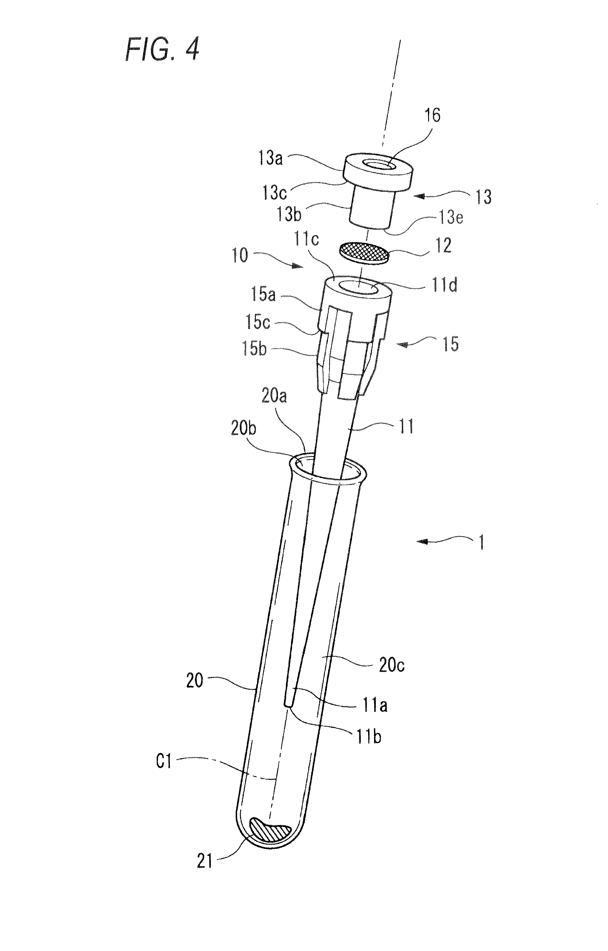

Fig. 4 shows a state where the cell isolation device 1 is disassembled. The cell isolation device 1 is configured by a pipette 10 and a container 20. The both components are formed by a resin material or the like which is noncytotoxic. The pipette 10 includes a body 11, a filter 12, and a lid member 13.

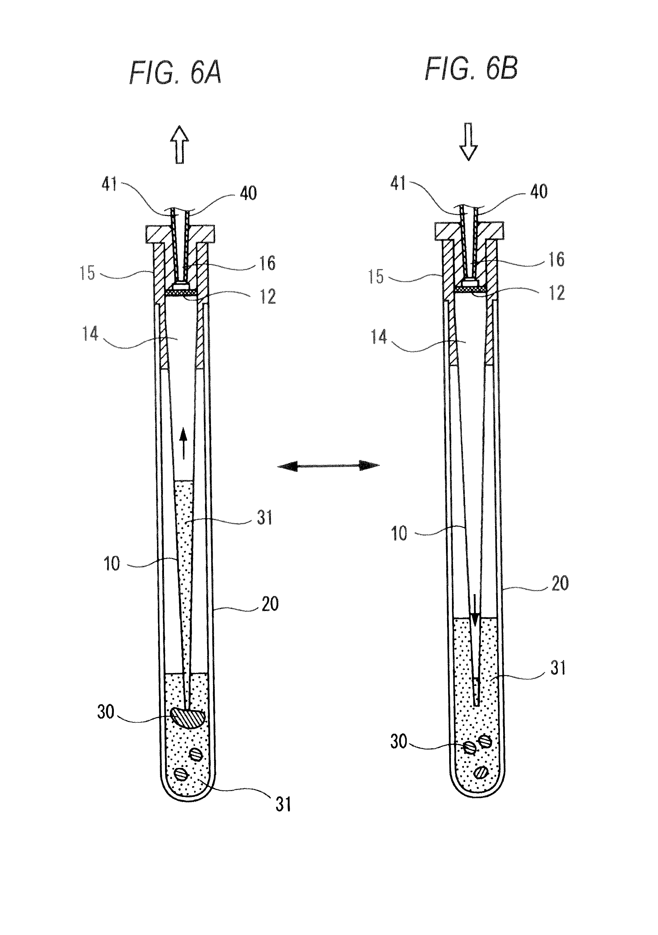

The body 11 is a hollow cylindrical member in which a tip end portion 11a has a tapered shape. An opening 11b is formed in the tip end (lower end) of the body 11 and an opening 11d is formed in the upper end surface 11c. The openings 11b, 11d communicate with each other through a passage 14 (see Figs. 6A and 6B) which is formed inside the body 11.

A holding member 15 is disposed in an upper end portion of the body 11. The holding member 15 has a large-diameter portion 15a and a small-diameter portion 15b. A step 15c is defined in the interface between the large-diameter portion 15a and the small-diameter portion 15b. The large-diameter portion 15a includes the upper end surface 11c.

The lid member 13 is a cylindrical member having a large-diameter portion 13a and a small-diameter portion 13b. A step 13c is defined in the interface between the large-diameter portion 13a and the small-diameter portion 13b. An opening is formed in the upper end surface including the large-diameter portion 13a, and an opening is formed in the lower end surface including the small-diameter portion 13b. The both openings communicate with each other through a passage 16 which is formed inside the lid member 13.

The filter 12 is formed by a material which is noncytotoxic, and has mesh openings which allow a liquid containing isolated cells (cells in which nuclei are isolated) to pass therethrough. In the embodiment, a nylon mesh having mesh openings of 50 µm is used as the filter 12, and adhered or welded to the lid member 13 so as to cover an opening 13e.

The filter 12 and the small-diameter portion 13b of the lid member 13 are inserted into the opening 11d to be attached to the body 11, and the step 13c of the lid member 13 is adhered or welded to the upper end surface 11c of the body 11. In this state, the passage 14 of the body 11 and the passage 16 of the lid member 13 communicate with each other through the filter 12.

The container 20 is a cylindrical member which has an opening 20b in the upper end surface 20a, and in which a lower end portion is configured as a round bottom. The container 20 is transparent so that the hollow internal space 20c is visible. The internal space 20c communicates with the opening 20b.

The pipette 10 is attached to the container 20 in which the tissue 30 to be subjected to cell isolation is accommodated in the internal space 20c. Specifically, the body 11 of the pipette 10 is inserted from the opening 20b of the container 20 into the internal space 20c until the upper end surface 20a of the container 20 abuts against the step 15c of the holding member 15. In this state, the outer circumferential surface of the small-diameter portion 15b of the holding member 15 abuts against the inner surface of the container 20.

Namely, the holding member 15 is fitted to an upper end portion of the container 20, so that a tip end portion 10a of the pipette 10 is placed in a predetermined position in the internal space 20c of the container 20. Specifically, the tip end (opening 11b) of the body 11 is placed on the central axis C1 of the container 20, and opposed to the bottom of the internal space 20c via a constant gap.

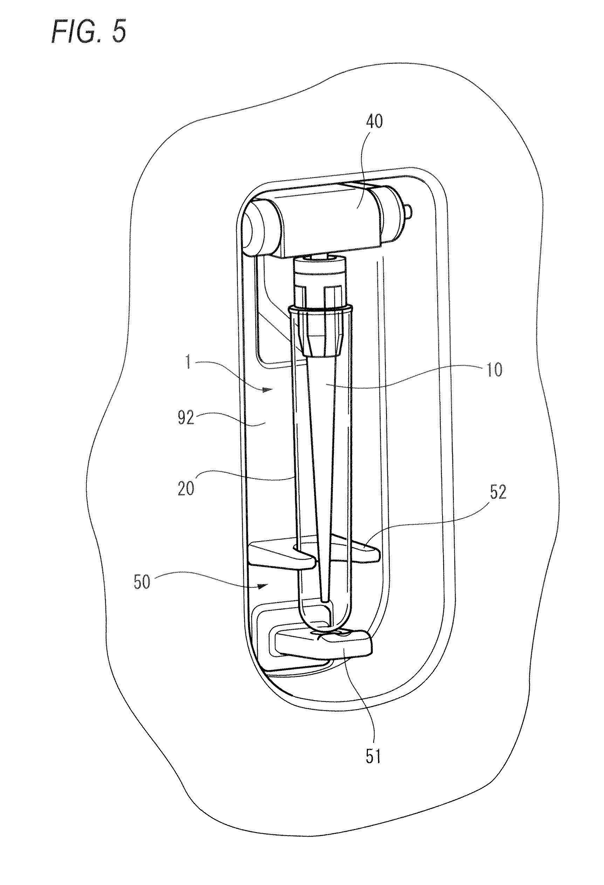

The thus configured cell isolation device 1 is attached to a tip end portion of the nozzle 40 shown in Fig. 2, and then the passage 16 of the lid member 13 and the passage 41 in the body housing 90 communicate with each other in an air- and liquid-tight manner. When the nozzle 40 is rearward swung in this state, the cell isolation device 1 is housed in the container housing 92 as shown in Fig. 5. At this time, the lower end portion of the container 20 is supported by the supporting table 51, and side portions of the container 20 are held by the holder 52, whereby the container 20 is prevented from being displaced.

When the user inputs instructions for starting the cell isolation process through the operating unit 94, the controller 80 controls the valve 43 so as to cause the passage 41 to communicate with the first branch passage 45. Then, the controller 80 controls the pump 42 so as to set the passage 41 to the pressurized condition, thereby causing the cell treatment solution 31 accommodated in the treatment solution accommodator 71 to be ejected from the nozzle 40.

The cell treatment solution 31 is injected into the internal space 20c of the container 20 through the passage 14 in the pipette 10. The injection amount is predetermined so that at least the tip end portion 11a of the pipette 10 dips into the injected cell treatment solution 31.

A reagent 21 containing a surfactant, an RNA (ribonucleic acid) remover, and a fluorescent dye/pigment is accommodated on the bottom of the internal space 20c in a state where the reagent is dried or freeze-dried. When the cell treatment solution 31 is loaded, the reagent 21 dissolves in the cell treatment solution 31.

Preferably, a solution in which the osmotic pressure is equal to that of a living body, such as PBS (phosphate buffer solution) is used as the cell treatment solution 31. In parallel with a below-described cell isolation process by pipetting, nuclei isolation of tissue cells by the surfactant, RNA removal by the RNA remover, and staining of isolated DNA cell nuclei by the fluorescent dye/pigment can be performed. This enables that, after recovery by the cell isolation apparatus which will be described later, measurement by a fluorescent analyzer (flow cytometer) or the like is performed. Therefore, rapid diagnosis can be realized.

Next, the controller 80 controls the valve 43 so as to set a state where both the first branch passage 45 and the second branch passage 46 do not communicate with the passage 41. Then, the controller 80 controls the pump 42 so as to alternately form the pressurized condition and the depressurized condition in the passage 41. In the pressurized condition, the air is blown out from the nozzle 40 through the passage 41, and, in the depressurized condition, the air is sucked from the nozzle 40 through the passage 41.

Namely, the controller 80 controls the operation of the pump 42 so as to eject the air from the nozzle 40 or so as to suck the air into the nozzle 40, thereby causing the pipette 10 to pipet the tissue 30 and the cell treatment solution 31 (containing the reagent 21) in the container 20.

Fig. 6A shows a state where the depressurized condition is formed. A predetermined suction force acts on the pipette 10, and the cell treatment solution 31 in the container 20 is sucked into the pipette 10. Part of the cell treatment solution 31 is raised in the passage 41, and the tissue 30 is attracted to the tip end (opening 11b) of the pipette 10. At this time, part of the tissue is smashed by collision with the tip end of the pipette 10.

Fig. 6B shows a state where the pressurized state is formed. A predetermined pressure is applied to the pipette 10, and the cell treatment solution 31 in the passage 41 is ejected from the opening 11b to be returned to the internal space 20c of the container 20. At this time, the tissue 30 which is attracted to the opening 11b is returned into the cell treatment solution 31 while part of the tissue is smashed by shock caused by the ejection.

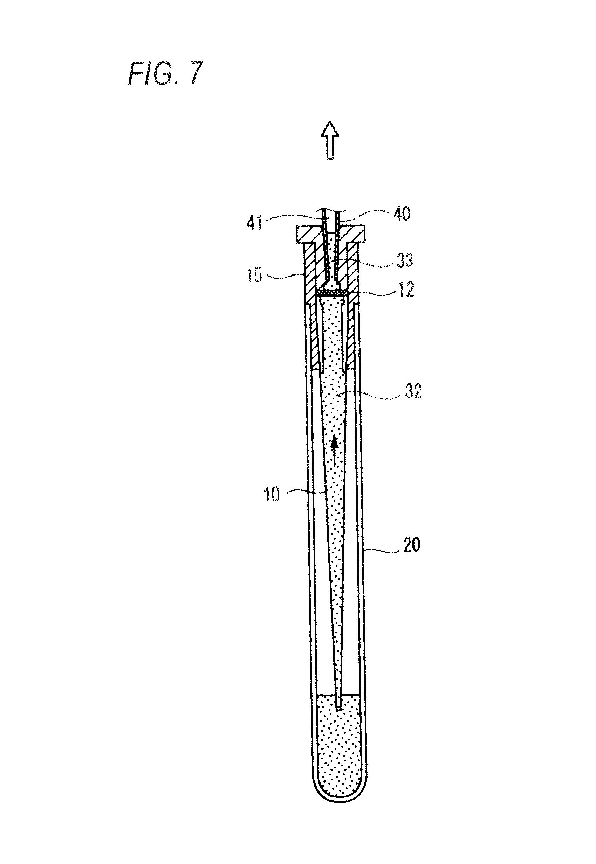

By repeating the above-described suction and ejection processes, the tissue 30 is gradually finely smashed to enter a minced state. When the pipetting process is performed for a predetermined time period, a suspending solution 32 (see Fig. 7) containing isolated cells can be obtained.

The isolated cells are used in a pathological analysis. In addition to the isolated cells, however, unwanted minced tissue pieces are suspended in the suspending solution 32. In order to use only the isolated cells in the analysis, a step of filtering out tissue pieces which are larger than the isolated cells is necessary.

Therefore, the controller 80 controls the valve 43 so as to cause the passage 41 to communicate with the second branch passage 46. Then, the controller 80 controls the pump 42 so as to set the passage 41 to the depressurized condition where the degree of the depressurization is higher than that in the pipetting process, thereby causing a suction force which is larger than that in the pipetting process, to act on the pipette 10.

As shown in Fig. 7, then, the suspending solution 32 in the container 20 is sucked into the pipette 10, and raised in the passage 14. When the sucking operation of the pump 42 is continued, the suspending solution 32 reaches the filter 12 which separates the passage 14 from the passage 16. When the suspending solution 32 passes through the filter 12, unwanted tissue pieces are filtered out, and a cell suspension 33 containing desired isolated cells is obtained in the passage 16. When the sucking operation is further continued, the cell suspension 33 is recovered to the recovered solution accommodator 72 after passing through the passage 41 and the second branch passage 46. The recovered cell suspension 33 is then used in an analyzing process such as a fluorescence analysis by using a flow cytometer.

The passage 41 which is used in both the supply of the cell treatment solution and the recovery of the cell suspension has a structure in which the cell suspension is not diluted with the cell treatment solution. For example, such a structure may be obtained by, for example, making the length of the passage 41 as short as possible, making the diameter of the passage 41 as small as possible, forming the passage 41 by a water-repellant material such as Teflon (registered trademark), or coating the inner surface of the passage with such a material.

Namely, the injection of the cell treatment solution 31 into the cell isolation device 1, the cell isolation process due to pipetting, and the process of recovering isolated cells by filtering the suspending solution 32 can be executed while the same cell isolation device 1 remains to be connected to the nozzle 40. Furthermore, the steps can be automatized, and hence the working efficiency can be remarkably improved.

The tissue 30 to be used in the cell isolation process is different in hardness and the like depending on the body portion from which the tissue is harvested. Even when cell isolation processes are performed under constant conditions, results of the cell isolation processes may be different from each other. Therefore, the cell isolation apparatus 100 of the embodiment is configured so that the user can input and set conditions of the operation control of the pump 42 which is performed by the controller 80.

Specifically, control conditions are input through the operating unit 94 which functions as the condition inputting unit in the presently disclosed subject matter. As the control conditions, the rate of ejection of the air from the nozzle 40, the rate of suction of the air into the nozzle, the amount of ejection of the air, the amount of suction of the air, the duration time of ejection of the air, the duration time of suction of the air, the interval between ejection and suction, and the ejection and suction numbers are made variable.

According to the configuration, under a plurality of different conditions, a pipetting process can be executed on the tissue 30 which is harvested under certain conditions, to obtain optimum control conditions for attaining a satisfactory result of cell isolation. In the case where the cell isolation process is performed on the tissue 30 in which such optimum conditions have been known, conditions for the pipetting process are set through the operating unit 94 so as to coincide with the optimum conditions, whereby a satisfactory result of cell isolation can be attained. Therefore, constant results of cell isolation can be attained automatically an efficiently without performing a procedure that depends on the skill level.

When hard tissue is drawn into the pipette 10, it is often that the tissue is not sufficiently smashed by the tip end portion, and clogging easily occurs. In such a case, depressurization is excessively performed by the pump 42, and this may cause a failure or the like.

In the embodiment, therefore, a third branch passage 47 extends from a part of the passage 41 to be connected to a pressure sensor 61 as shown in Fig. 3. Namely, the embodiment is configured so that the internal pressure of the pipette 10 is sensed by the pressure sensor 61 via the nozzle 40.

The pressure sensor 61 and the controller 80 are communicatably connected to each other, and the internal pressure of the pipette 10 is monitored by the controller 80. In the case where the pipette 10 is clogged with the tissue 30, for example, the internal pressure is abnormally raised, and therefore the controller 80 can surely know the situation. At this time, the controller 80 execute a process of eliminating clogging.

In the process of eliminating clogging, specifically, the controller 80 once stops the operation of the pump 42 to eliminate the excessively depressurized condition. Then, the operation of the pump 42 is controlled so that the passage 41 enters a slightly highly pressurized condition, thereby removing the tissue 30 clogging the pipette 10. After elapse of a constant time period, the predetermined pipetting process is restarted.

In the case where clogging with the tissue 30 frequently occurs, or in the case where a clogged state is not canceled even by the above-described process of eliminating clogging, the controller 80 outputs a visual or auditory alarm through the display of the operating unit 94 or the like to prompt the user to change the pipetting conditions or replace the specimen. Alternatively, the pipetting process may not be reexecuted, and an alarm may be output at the timing when the operation of the pump 42 is stopped.

The user resets the pipetting conditions through the operating unit 94. For example, a change in which the duration time is shortened and the numbers of ejections and suctions are increased by increasing the air ejection/suction rates may be performed. According to the configuration, isolation can be surely performed without imposing an undue burden on tissue. Moreover, a trouble caused by a phenomenon that the pipette 10 is clogged with the tissue 30 can be prevented from occurring, and, with respect to the tissue 30, more appropriate pipetting conditions can be set.

As shown in Fig. 3, a container sensor 62 which senses whether the container 20 of the cell isolation device 1 is supported or not is disposed in the supporting table 51 of the supporting portion 50. The container sensor 62 is communicatably connected to the controller 80.

In the embodiment, the container sensor 62 is configured by a pressure sensor. When, as shown in Fig. 5, the cell isolation device 1 is housed in the container housing 92 and the lower end portion of the container 20 is contacted with the supporting table 51, the container sensor 62 detects the pressure and outputs a detection signal to the controller 80.

The controller 80 is configured so as not to cause the pump 42 and the valve 43 to operate unless the controller 80 receives the detection signal. According to the configuration, it is possible to prevent a trouble in which, in the state where the cell isolation device 1 is not housed in the container housing 92, the cell treatment solution 31 is ejected from the nozzle 40 or the pump 42 performs an idle sucking operation.

As described with reference to Fig. 1, the light-shielding cover 93 is disposed in the front panel 91. The light-shielding cover 93 which functions as the light-shielding member in the presently disclosed subject matter covers the container 20 of the cell isolation device 1 supported by the supporting portion 50, in a position where the light-shielding cover closes the container housing 92 indicated by the dash-dot-dot line in Fig. 1. Therefore, fading of fluorescent dye/pigment contained in the reagent 21 can be prevented from occurring during the cell isolation process, and it is possible to secure the accuracy of fluorescence analysis in the subsequent stage.

As shown in Fig. 3, in the vicinity of the supporting portion 50 in the container housing 92, a temperature regulator 63 for regulating the temperature of the container housing 92 including the vicinity is disposed. The temperature regulator 63 has a related-art structure which is configured by a thermistor, a Peltier element, a cooling fan, and the like, and therefore its detailed description is omitted. A temperature sensor 64 is connected to the temperature regulator 63. The temperature regulator 63 and the temperature sensor 64 are communicatably connected to the controller 80.

The temperature sensor 64 outputs a signal indicative of the sensed temperature of the vicinity of the supporting portion 50, to the controller 80. According to the configuration, when the result of the cell isolation process is to be evaluated, the environmental temperature at which the process is performed may be added to evaluation parameters, and may be contributory to setting of the pipetting conditions.

The controller 80 is configured so as to control the operation of the temperature regulator 63 based on the temperature sensed by the temperature sensor 64, to maintain the temperature of the vicinity of the supporting portion 50 to a predetermined temperature. In the case where the cell treatment solution 31 contains an enzyme, for example, the temperature of the vicinity of the supporting portion 50 in which a reaction occurs can be maintained constant, and the reaction can proceed in a stable condition.

The embodiment has been described in order to facilitate understanding of the presently disclosed subject matter, and is not intended to limit the presently disclosed subject matter. It is a matter of course that the presently disclosed subject matter may be changed or improved without departing the spirit thereof, and includes equivalent embodiments.

The apparatus may be configured so that the controller 80 automatically changes the conditions of the operation control of the pump 42 related to the pipetting process, based on the internal pressure of the pipette 10 which is sensed by the pressure sensor 61.

The conditions which the user can change through the operating unit 94, or the controller 80 can automatically change are not required to be all of the rate of ejection of the air from the nozzle, the rate of suction of the air into the nozzle, the amount of ejection of the air, the amount of suction of the air, the duration time of ejection of the air, the duration time of suction of the air, the interval between ejection and suction, and the ejection and suction numbers. It is necessary only that at least one of these values is variable.

The container sensor 62 is not required to be a pressure sensor which is disposed in the supporting table 51. As far as the tissue 30, the cell treatment solution 31, and the reagent 21 are not optically affected, a configuration may be employed where a transmissive or reflective optical sensor is disposed in one of the supporting table 51 and the holder 52, and the sensor optically detects attachment of the container 20 to the supporting portion 50.

The operating unit 94 which functions as the condition inputting unit in the presently disclosed subject matter is not always required to be a man-machine interface disposed in the front panel 91. The controller 80 may be configured so as to be communicatable with an external computer, and the control conditions of the pump 42 for executing the pipetting process may be set or changed through an application operating on the computer.

When the automatization of injection of the cell treatment solution 31 is not required, the treatment solution accommodator 71 and the first branch passage 45 may be omitted. In this case, the configuration of the valve 43 is adequately changed.

When the automatization of recovery of the cell suspension 33 after the pipetting process is not required, the recovered solution accommodator 72 and the second branch passage 46 may be omitted. In this case, the configuration of the valve 43 is adequately changed.

The material, color, and shape of the light-shielding cover 93 which functions as the light-shielding member in the presently disclosed subject matter may be adequately selected so as to attain appropriate optical characteristics, in accordance with light-shielding conditions required in the cell isolation process. When light shielding during the cell isolation process is not necessary, the light-shielding cover 93 may be omitted.

At least one of the pressure sensor 61, the container sensor 62, the temperature regulator 63, and the temperature sensor 64 may be appropriately omitted in accordance with a request for the cost or the like.

According to an aspect of the presently disclosed subject matter, under a plurality of different conditions, a pipetting process can be executed on tissue which is harvested under certain conditions, and optimum control conditions for attaining a satisfactory result of cell isolation can be obtained. In the case where the cell isolation process is to be performed on tissue in which such optimum conditions have been known, conditions for the pipetting process are set through the condition inputting unit so as to coincide with the optimum conditions, whereby a satisfactory result of cell isolation can be obtained. Therefore, a constant result of cell isolation can be attained automatically and efficiently without performing a procedure that depends on the skill level.

When hard tissue is drawn into the pipette, it is often that the tissue is not sufficiently smashed by a tip end portion, and clogging easily occurs. In such a case, the internal pressure of the pipette is abnormally raised. According to the an aspect of the present disclosed subject matter, since a pressure sensor which is configured to sense an internal pressure of the pipette through the nozzle is provided, it is possible to surely know this situation.

According to the an aspect of the present disclosed subject matter, since the condition of the control is changed based on the internal pressure, isolation can be surely performed without imposing an undue burden on tissue. Moreover, a trouble caused by a phenomenon that the pipette is clogged with tissue, such as a failure of the pump can be prevented from occurring, and, with respect to the tissue, more appropriate pipetting conditions can be set.

According to the an aspect of the present disclosed subject matter, since the operation of the pump is controlled to eject the cell treatment solution which is accommodated in the treatment solution accommodator, from the nozzle, injection of the cell treatment solution into the container, and the cell isolation process due to pipetting can be executed while the same pipette remains to be connected to the nozzle. Furthermore, steps can be further automatized, and hence the working efficiency can be remarkably improved.

According to the an aspect of the present disclosed subject matter, since the operation of the pump is controlled to cause a cell suspension which is obtained by the pipetting, to be recovered from the nozzle, and the recovered cell suspension to be accommodated in the recovered solution accommodator, the cell isolation process due to pipetting, and a process of recovering isolated cells by filtering a suspending solution can be executed while the same pipette remains to be connected to the nozzle. Furthermore, steps can be further automatized, and hence the working efficiency can be remarkably improved.

According to an aspect of the presently discloses subject matter, since a supporting portion which supports the container accommodating the tissue and the fluid, and a container sensor which is configured to sense whether the container is supported by the supporting portion or not are further provided, the apparatus can be configured so that the pump does not operate unless the container sensor outputs the detection signal. Therefore, a trouble in which, in a state where the pipette is not attached to the nozzle, the cell treatment solution is ejected from the nozzle, or the pump performs an idle sucking operation can be prevented from occurring.

According to the an aspect of the present disclosed subject matter, since a light-shielding member which covers the container supported by the supporting portion is provided, fading of fluorescent dye/pigment contained in a reagent can be prevented from occurring during, for example, the cell isolation process, and it is possible to secure the accuracy of fluorescence analysis by a cell analyzing apparatus such as a flow cytometer.

According to the an aspect of the present disclosed subject matter, since a temperature sensor which is disposed in a vicinity of the supporting portion is provided, when a result of the cell isolation process is to be evaluated, the environmental temperature at which the process is performed may be added to evaluation parameters, and may be contributory to setting of the pipetting conditions.

According to the an aspect of the present disclosed subject matter, since a temperature regulator which is configured to regulate a temperature of the vicinity of the supporting portion, and an operation of the temperature regulator is controlled based on a temperature which is sensed by the temperature sensor, in the case where the cell treatment solution contains an enzyme, for example, the temperature of the vicinity of the supporting portion in which a reaction occurs can be maintained constant, and the reaction can proceed in a stable condition.

## Claims

1. A cell isolation apparatus in which tissue and fluid that are accommodated in a container are pipetted by a pipette, and cells are isolated from the tissue, the cell isolation apparatus comprising:
a nozzle to which the pipette is attached;
a pump which is connected to the nozzle;
a controller which is configured to control an operation of the pump to cause air to be ejected from the nozzle or to be sucked into the nozzle, thereby causing the pipette to perform pipetting; and
a condition inputting unit in which a user inputs a condition of control of the controller,
as the condition of the control, at least one of a rate of ejection of air from the nozzle, a rate of suction of air into the nozzle, an amount of suction of air, an amount of ejection of air, a duration time of ejection of air, a duration time of suction of air, an interval between ejection and suction, and ejection and suction numbers being variable.

2. The cell isolation apparatus according to claim 1, further comprising a pressure sensor which is configured to sense an internal pressure of the pipette through the nozzle.

3. The cell isolation apparatus according to claim 2, wherein the controller changes the condition of the control based on the internal pressure which is sensed by the pressure sensor.

4. The cell isolation apparatus according to any one of claims 1 to 3, further comprising a treatment solution accommodator which is configured to accommodate a cell treatment solution, wherein
the controller controls the operation of the pump to eject the cell treatment solution which is accommodated in the treatment solution accommodator, from the nozzle.

5. The cell isolation apparatus according to any one of claims 1 to 4, further comprising a recovered solution accommodator which is configured to accommodate a cell suspension, wherein
the controller controls the operation of the pump to cause a cell suspension which is obtained by the pipetting, to be recovered from the nozzle, and the recovered cell suspension to be accommodated in the recovered solution accommodator.

6. The cell isolation apparatus according to any one of claims 1 to 5, further comprising:
a supporting portion which supports the container accommodating the tissue and the fluid; and
a container sensor which is configured to sense whether the container is supported by the supporting portion or not.

7. The cell isolation apparatus according to claim 6, further comprising a temperature sensor which is disposed in a vicinity of the supporting portion.

8. The cell isolation apparatus according to claim 7, further comprising a temperature regulator which is configured to regulate a temperature of the vicinity of the supporting portion, wherein
the controller controls an operation of the temperature regulator based on a temperature which is sensed by the temperature sensor.

9. The cell isolation apparatus according to claim 6, further comprising a light-shielding member which covers the container supported by the supporting portion.

10. The cell isolation apparatus according to claim 9, further comprising a temperature sensor which is disposed in a vicinity of the supporting portion.

11. The cell isolation apparatus according to claim 10, further comprising a temperature regulator which is configured to regulate a temperature of the vicinity of the supporting portion, wherein
the controller controls an operation of the temperature regulator based on a temperature which is sensed by the temperature sensor.
